(19) Europäisches Patentamt
European Patent Office
Office européen des brevets

(11) Veröffentlichungsnummer: **0 300 387 B1**

(12) **EUROPÄISCHE PATENTSCHRIFT**

(45) Veröffentlichungstag der Patentschrift: **23.03.94**

(51) Int. Cl.5: **C07D 209/48**, A01N 37/32, C07C 205/56, C07C 229/44

(21) Anmeldenummer: **88111455.7**

(22) Anmeldetag: **16.07.88**

Die Akte enthält technische Angaben, die nach dem Eingang der Anmeldung eingereicht wurden und die nicht in dieser Patentschrift enthalten sind.

# (54) Phenylalkenylcarbonsäuren und deren Ester.

(30) Priorität: **23.07.87 DE 3724399**

(43) Veröffentlichungstag der Anmeldung:
**25.01.89 Patentblatt 89/04**

(45) Bekanntmachung des Hinweises auf die Patenterteilung:
**23.03.94 Patentblatt 94/12**

(84) Benannte Vertragsstaaten:
**BE CH DE FR GB IT LI**

(56) Entgegenhaltungen:
**EP-A- 0 240 659**
**DE-A- 2 210 672**

**CHEMICAL ABSTRACTS, Band 105, 1986, Seite 606, Zusammenfassung Nr. 60524v, Columbus, Ohio, US; S. MATSUMOTO et al.: "Herbicidal N-substituted dicarboxyimides"**

**CHEMICAL ABSTRACTS, Band 102, 1985, Zusammenfassung Nr. 24478n, Columbus, Ohio, US; "4,5,6,7-Tetrahydro-2H-isoindole-1,3-diones"**

(73) Patentinhaber: **BASF Aktiengesellschaft**
**Carl-Bosch-Strasse 38**
**D-67063 Ludwigshafen(DE)**

(72) Erfinder: **Plath, Peter**
**Hans-Balcke-Strasse 13**
**D-6710 Frankenthal(DE)**
Erfinder: **Eicken, Karl**
**Am Huettenwingert 12**
**D-6706 Wachenheim(DE)**
Erfinder: **Goetz, Norbert, Dr.**
**Schoefferstrasse 25**
**D-6520 Worms 1(DE)**
Erfinder: **Wild, Jochen, Dr.**
**An der Marlach 7**
**D-6705 Deidesheim(DE)**
Erfinder: **Meyer, Norbert, Dr.**
**Dossenheimer Weg 22**
**D-6802 Ladenburg(DE)**
Erfinder: **Wuerzer, Bruno, Dr.**
**Ruedigerstrasse 13**
**D-6701 Otterstadt(DE)**

**EP 0 300 387 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

**CHEMICAL ABSTRACTS, Band 79, 1973, Seiten 288,289, Zusammenfassung Nr. 41607c, Columbus, Ohio, US; E.R. KRAJNIAK et al.: "Tropolones and other products from a Reimer-Tiemann reaction"**

## Beschreibung

Die vorliegende Erfindung betrifft in 5-Stellung durch Tetrahydro-2H-isoindol-1,3-dion (Tetrahydrophthalimid) substituierte Phenylalkenylcarbonsäuren und deren Ester, ein Verfahren zu ihrer Herstellung sowie deren Verwendung als Herbizide.

Es ist bekannt, daß bestimmte isoindoldionsubstituierte Zimtsäureester als Herbizide verwendet werden können. Diese aus der JP-OS-59/115 358 bekannten Verbindungen haben die allgemeine Formel Z

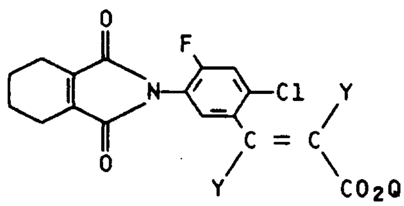

Z

in der Y Wasserstoff oder Methyl und Q eine Alkylgruppe, besonders Ethyl, bedeuten. Ferner ist aus EP-OS 68 822 die Struktur $Z^1$ bekannt:

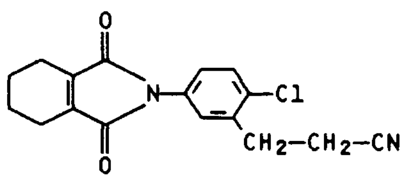

$Z^1$

Ihre herbizide Wirkung und die Verträglichkeit gegenüber Kulturpflanzen befriedigen in der Praxis jedoch keineswegs.

Die Erfindung hatte daher zum Ziel, besser verträgliche und zugleich gegen Unkraut wirksamere Herbizide bereitzustellen. Die erfindungsgemäßen Verbindungen haben die allgemeine Formel I

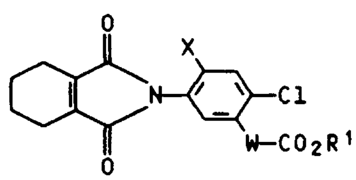

I

in der bedeuten:

- X Wasserstoff oder Fluor
- $R^1$ Wasserstoff, $C_1$-$6_6$-Alkyl, $C_5$- oder $C_6$-Cycloalkyl, ($C_1$-$C_4$-Alkoxy)-$C_2$-$C_4$-alkyl oder ($C_1$-$C_4$-Alkylthio)-$C_2$-$C_4$-alkyl;
- W den zweiwertigen Rest $-CH=CR^4-CH_2-$; $-CH=CR^2-CH=CR^3-$ oder

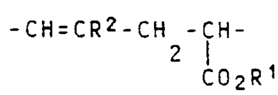

wobei

$R^2$, $R^3$ für Wasserstoff oder $C_1$-$C_4$-Alkyl und $R^4$ für $C_1$-$C_4$-Alkyl stehen und zusätzlich X Fluor und W die Gruppe $-CH=CY-$ bedeuten, wobei Y für Chlor oder Brom steht.

Die neuen Stoffe (I) eignen sich z.B. vorzüglich zur Unkrautbekämpfung in Weizen, Mais, Reis und Soja.

Die Stoffe der Formel I werden z.B. dadurch erhalten, daß man ein Anilin der Formel II mit dem Anhydrid III in einem geeigneten Lösungsmittel bei 40 bis 120 °C in einem Zeitraum von i.a. nicht mehr als 24 Stunden umsetzt (Schema 1):

Schema 1:

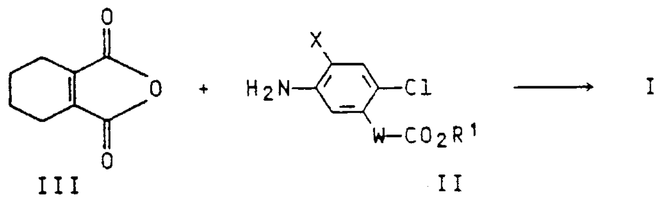

III II

Die Umsetzung wird zweckmäßig in einem geeigneten Lösungsmittel wie einem aliphatischen Kohlenwasserstoff (z.B. Hexan oder Ligroin), aromatischen Kohlenwasserstoff (z.B. Toluol, Xylol), Ether (z.B. Tetrahydrofuran, Diethylenglykoldimethylether), einer niederen Fettsäure (z.B. Essigsäure, Propionsäure) und Mischungen der letzteren mit Wasser vorgenommen. Das Reaktionsprodukt wird in üblicher Weise durch Extraktion, Ausfällung und wenn nötig anschließende Umkristallisation isoliert und, falls erforderlich, durch Chromatografie gereinigt.

Das Anhydrid III ist ein bekanntes Zwischenprodukt. Aniline der Formel II werden dadurch erhalten, daß man eine entsprechende Nitroverbindung katalytisch hydriert oder durch ein geeignetes Reduktionsmittel (z.B. Eisen in Methanol/Eisessig) zu II reduziert (Schema 2):

Schema 2:

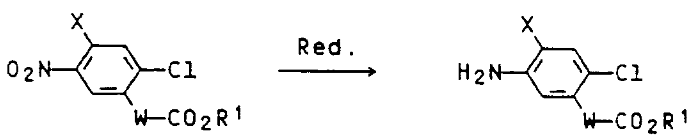


IV II

Die Nitroverbindungen IV können auf bekannte Weise erhalten werden, wobei man verschiedene Methoden verwenden kann, je nachdem, welche Bedeutung dem Rest W zukommt:

1. Wenn W für den (halogenierten) Rest -CH = CY- steht, wird die benötigte Nitroverbindung (8) entweder durch Halogenierung des Esters (1), nachfolgende Dehydrohalogenierung sowie anschließende Nitrierung hergestellt (Schema 3), oder durch Halogenierung und Dehydrohalogenierung eines Nitrozimtsäureesters (2b), wie es in Schema 4 skizziert ist:

Schema 3:

(1) 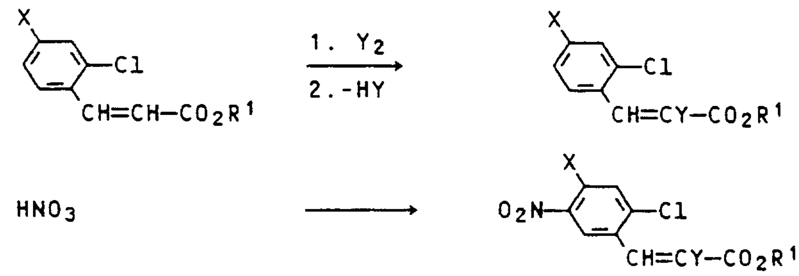

(7)

(7) + $HNO_3$ (8)

Schema 4:

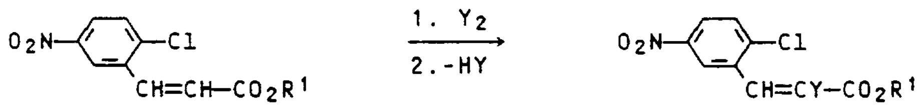

(2b) (8a)

Die 2-Chlor-5-nitrozimtsäure (2a) wird direkt durch Umsetzung von Nitrobenzaldehyd (6) mit Malonsäure erhalten (Schema 5):

Schema 5:

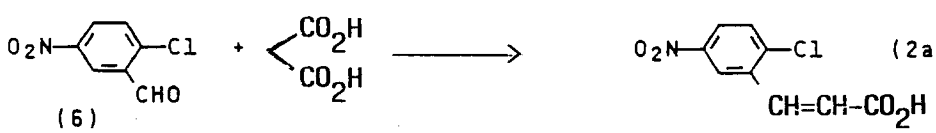
(6) (2a)

Durch Veresterung von 2a wird der entsprechende Zimtsäureester 2b erhalten.

Der bevorzugte Weg zur Herstellung einer Verbindung Ib mit W = $-CH=CY-CO_2R^1$ führt jedoch über die Halogenierung und Dehydrohalogenierung der Struktur Ia:

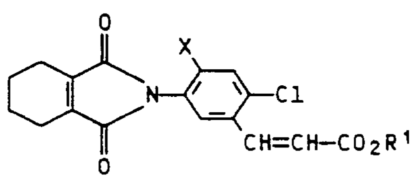
Ia

Während die Bromaddition an Ia (Y = Br) problemlos gelingt, müssen zur Chloraddition Maßnahmen ergriffen werden, die bei den Beispielen beschrieben werden.

Die zur Dehydrohalogenierung benötigten Basen sind beispielsweise Triethylamin, Pyridin oder $\alpha$-Picolin.

Als Lösungsmittel dient bei der Halogenaddition bevorzugt 1,1,1-Trichlorethan oder Eisessig, während die Dehydrohalogenierung in Methylenchlorid, Essigsäureethylester, 1,1,1-Trichlorethan o.ä. durchgeführt wird.

Die Nitroverbindung (2) zur Herstellung von Ia erhält man beispielsweise durch Nitrierung des bekannten $\alpha$-Alkyl-Zimtsäureesters (1) gemäß Schema (6):

**Schema 6:**

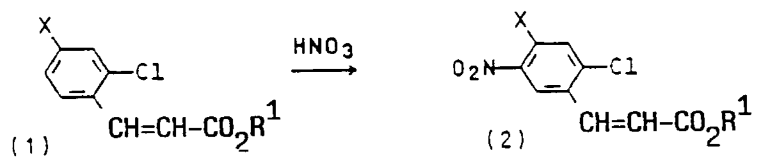


(1) (2)

Die Nitrierung erfolgt für X=H vorteilhaft mit 96 - 100 %iger Salpetersäure bei -10°C bis 0°C. Die entsprechende Fluorverbindung kann auch in konzentrierter Schwefelsäure als Verdünnungsmittel nitriert werden.

Der Ester (1) wird in üblicher Weise durch Veresterung der Säure (5) erhalten, die aus dem Benzaldehyd (3) durch Umsetzung mit Malonsäure hergestellt wird (Schema 7):

**Schema 7:**

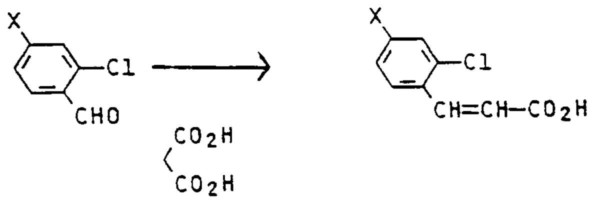

(5)

Knoevenagel-Kondensation

Die zur Herstellung von (5) geeigneten Bedingungen sind bekannt; über die Knoevenagel-Kondensation wird in Org. Reactions 15, 204 (1967) zusammenfassend berichtet.

2. Wenn W eine Kette aus mehr als zwei Kohlenstoffatomen enthält, richtet sich das Verfahren zur Herstellung der erforderlichen Nitroverbindung danach, ob X Fluor oder Wasserstoff bedeutet:

2.1 Wenn X Wasserstoff bedeutet, geht man entweder von einem Nitrozimtaldehyd (9) oder von dem Nitrobenzaldehyd (6) aus. Die erforderliche Kohlenstoffkette W wird dann dadurch erhalten, daß (9) entweder mit Malonsäure in Pyridin oder mit dem Oxazolin (10) in Toluol umgesetzt oder einer Perkin-Kondensation mit dem Anhydrid (4) unterworfen wird.

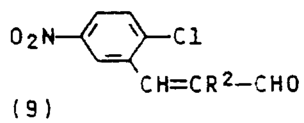

(9)

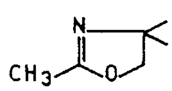

(10)

Alternativ gelingt der Aufbau der Kohlenstoffkette W durch Umsetzung von (6) mit dem Phosphonester (11) oder von (9) mit dem Phosphonester (12) in Gegenwart von Natriumhydrid in Dioxan oder Dimethylformamid (Horner-Emmons-Reaktion).

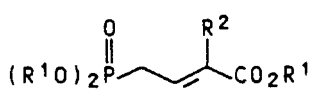

(11)

$(CH_3)_2N{-}CH\left[PO(OR^1)_2\right]_2$

(12)

(12) wird nach den Angaben in Angew. 80, 364 (1968) hergestellt.

2.2 Wenn hingegen X Fluor bedeutet, wird zuerst die Kohlenstoffkette durch Knoevenagel-Kondensation, oder Horner-Emmons-Reaktion aus dem Benzaldehyd (13) bzw. dem Zimtaldehyd (14)

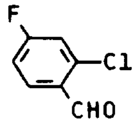 (13)

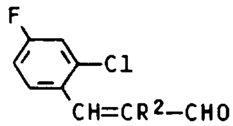 (14)

synthetisiert und danach die Nitrogruppe eingeführt.

In den angeführten Beispielen werden weitere Einzelheiten zum Aufbau einer Kette mit mehr als 2 C-Atomen mitgeteilt.

4. Wenn schließlich W für den Rest

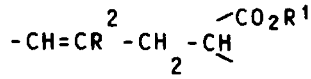

steht, kann das erforderliche Anilinderivat IIIa durch Umsetzung eines entsprechenden Nitro-cinnamyliden-malonesters mit $NaBH_4$ in Gegenwart von Kupfer-II-acetat erhalten werden:

Ein typisches Beispiel zur Herstellung einer Verbindung I ist das folgende:

Beispiel 1

Zielverbindung

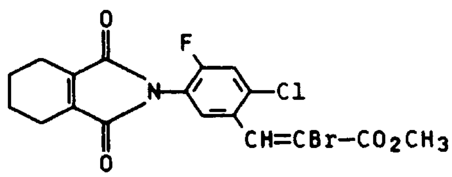

1.1 Analog dem in JP-OS-59/115 358 beschriebenen Verfahren wurde zunächst 2-Chlor-4-fluor-5-N-(3,4,5,6-tetrahydrophthalimido)zimtsäuremethylester hergestellt.

1.2 Zu einer Lösung von 10 g des Zimtsäuremethylesters in 100 ml $CH_2Cl_2$ werden 5 g Brom, gelöst in 20 ml $CH_2Cl_2$, getropft und danach noch eine Stunde zum Sieden erhitzt. Man läßt auf 25 °C abkühlen, fügt 8 ml Triethylamin zu und läßt 14 Std. bei 25 °C rühren. Man extrahiert 2 x mit 50 ml Wasser, trocknet und engt ein. Der Rückstand wird in Toluol gelöst und über Kieselgel chromatografiert. Nach Abziehen des Toluols verbleibt ein Feststoff (Fp. 121 - 123 °C; Ausbeute 9,5 g, d.h. 78 %; Wirkstoff Nr. 1-1 in Tabelle 1). Die übrigen Verbindungen dieser Tabelle wurden durch entsprechende Abwandlung aus anderen Ausgangsstoffen erhalten.

Beispiel 2

Zielverbindung: Wirkstoff 2-1 in Tabelle 2

2.1 4-(2-Chlor-5-nitrophenyl)-3-methyl-3-buten-säure

Zu einer Suspension von 0,6 g Natriumhydrid in 25 ml absolutem Dioxan wird unter leichter Kühlung (23 - 26 °C) eine Lösung von 6,6 g Tetraethyl-dimethylamino-methylendiphosphonat in 25 ml Dioxan getropft und eine Stunde gerührt. Danach wird eine Lösung von 4,5 g 3-(2-Chlor-5-nitrophenyl)-2-methyl-2-propenal in 25 ml Dioxan zugetropft und anschließend 12 Std. auf 50 °C erwärmt. Danach wird das Lösungsmittel am Rotationsverdampfer entfernt, der Rückstand in Diethylether (150 ml) gelöst und zweimal mit Wasser ausgeschüttelt. Nach Trocknen und Einengen verbleiben 6,8 g (84 %) einer öligen Flüssigkeit der Struktur:

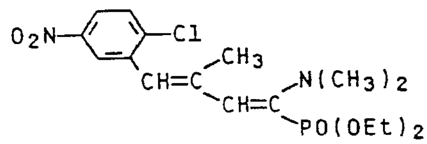

Durch einstündiges Kochen dieses Enamins mit 200 ml konz. HCl, Eingießen in 1 l Eiswasser und Extraktion mit Diethylether wurden nach Trocknen und Einengen 4,4 g Flüssigkeit erhalten.

2.2 4-(2-Chlor-5-nitrophenyl)-3-methyl-3-butensäure-methylester

4,3 g der nach 2.1 erhaltenen Säure werden bei Raumtemperatur in 60 ml Methanol gelöst und mit HCl-Gas gesättigt, wobei die Temperatur bis etwa 29 °C ansteigt. Nach 16 Std. Stehen wird das Methanol abdestilliert, der Rückstand in Diethylether gelöst, mit wäßrigem Na-hydrogencarbonat und danach mit Wasser gewaschen. Man trocknet mit $MgSO_4$ und dampft ein, wobei 3,3 g (72 %) des gewünschten Esters erhalten werden.

2.3 4-(2-Chlor-5-aminophenyl)-3-methyl-3-butensäuremethylester

Zu einer Suspension von 4,1 g Eisenpulver in einem auf 60 °C erhitzten Gemisch aus 34 ml Methanol und 9 ml Eisessig tropft man eine Lösung von 3,3 g des unter 2.2 erhaltenen Nitroaryl-butensäuresters in einer Mischung aus je 17 ml Methanol und Eisessig. Nach 2 Std. Erhitzen zum Sieden wird filtriert, das Filtrat fast zur Trockene eingedampft und mit 150 ml Essigsäureethylester ausgerührt. Nach Waschen der Lösung mit Wasser, Trocknen und Einengen erhält man 3,2 g eines Öls.

2.4 2-{4-Chlor-3-[(2'methyl-3'-methoxycarbonyl-1'-propenyl)-phenyl]}-4,5,6,7-tetrahydro-2H-isoindol-1,3-dion

3,0 g des nach 2.2 erhaltenen Anilinderivates und 1,9 g Tetrahydrophthalsäureanhydrid werden in 20 ml Eisessig gelöst und 2 Std. zum Sieden erhitzt. Danach wird das Lösungmittel abgedampft, in Essigsäureethylester gelöst, mit wäßrigem Na-bicarbonat und danach mit Wasser gewaschen, getrocknet und eingeengt. Der ölige Rückstand wird an Kieselgel mit Hexan/Aceton = 95 : 5 chromatografiert. Es verbleiben schließlich 1,4 g des Produktes als Öl, $n_D^{24}$ = 1,5579.

(Wirkstoff 2-2 in Tabelle 2; weitere Verbindungen auf entsprechende Weise)

Beispiel 3

Zielverbindung:

2-{4-Chlor-3-[(4'-methoxycarbonyl-1',3'-butadienyl)-phenyl]}-4,5,6,7-tetrahydro-2H-isoindol-1,3-dion (Wirkstoff 3-1 in Tabelle 3)

3.1 5-(2'-Chlor-5'-nitro-phenyl)-2,4-pentadiensäure

Zu einer Lösung von 6,05 g Malonsäure in 20 ml Pyridin werden bei 25 °C 10,15 g 2-Chlor-5-nitrozimtaldehyd und danach 0,45 g Piperidin gegeben. Anschließend wird 3 Std. zum Sieden erhitzt. Nach dem Abkühlen wird in eine Lösung von 20 ml konz. HCl in 50 ml Eiswasser gegossen, worauf das Produkt ausfällt. Nach Absaugen, Waschen mit Wasser und Trocknen i.V. bei 50 °C wird das Rohprodukt (11,9 g) in Toluol/Aceton = 8 : 2 an Kieselgel chromatografiert, wobei 5,6 g der gewünschten Säure als weißer Feststoff erhalten werden.

3.2 5-(2'-Chlor-5'-nitro-phenyl)-2,4-pentadiensäure-methylester

Ein Lösung von 5,4 g der nach 3.1 erhaltenen Carbonsäure in 60 ml Methanol wird mit HCl-Gas gesättigt und 16 Std. stehen gelassen. Nach Abdampfen des Lösungsmittels wird der Rückstand mit $NaHCO_3$-Wasser ausgerührt, abgesaugt und mit Wasser nachgewaschen. Nach Trocknen i. V. bei 50 °C erhält man 3,5 g des Esters (Fp. 132 °C; 62 % d.Th.).

3.3 5-(2'-Chlor-5'-aminophenyl)-2,4-pentadien-säuremethylester

Zu einer auf 60 °C erwärmten Suspension von 4,3 g Eisenpulver in 35 ml Methanol gießt man 9 ml Eisessig und tropft abschließend eine Lösung von 3,4 g des unter 3.2 dargestellten Esters in einer Mischung aus je 17 ml Methanol und Eisessig zu. Nach 1 Std. Kochen am Rückfluß wird eingedampft, mit Essigsäureethylester ausgerührt, mit Wasser gewaschen, getrocknet und eingeengt. Man erhält 2,9 g (96 % d.Th.) eines fablosen Öls.

3.4 Umsetzung mit 3,4,5,6-Tetrahydrophthalsäureanhydrid

2,2 g Tetrahydrophthalsäureanhydrid und 2,9 g des nach 3.3 erhaltenen Anilinderivates werden in 25 ml Eisessig gelöst und zwei Stunden bei 40 °C, danach 2 Std. bei 110 °C gerührt. Nach Abdampfen des Lösungsmittels wird der ölige Rückstand in Hexan gelöst und über Kieselgel chromatografiert.

Man erhält 2,3 g (59 % d. Th.) einer Verbindung, die bei 127 bis 130 ° C schmilzt. Nach NMR-Analyse liegt ein Gemisch folgender Stereoisomeren vor:

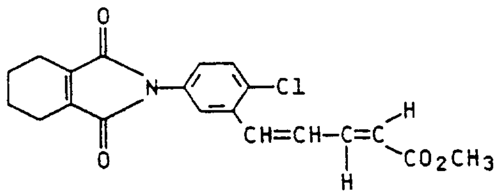

(45 % trans)

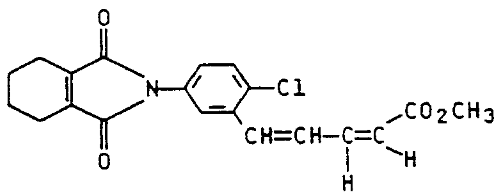

(55 % cis)

Beispiel 4

Zielverbindung:

2-{4-Chlor-3-[(4'-methoxycarbonyl-2'-methyl-1',3'-butadienyl)-phenyl]}-4,5,6,7-tetrahydro-2H-isoindol-1,3-dion (Wirkstoff 3-2 in Tabelle 3)

4.1 5-(2'-Chlor-5'-nitro-phenyl)-4-methyl-2,4-pentadiensäure
Eine Mischung aus 9 g 2-Chlor-5-nitro-α-methylzimtaldehyd und 10,4 g 2,4,4-Trimethyl-oxazolin in 50 ml Toluol wurde mit 0,3 g einer 40 proz. $NaHSO_3$-Lösung versetzt und 10 Std. am Rückfluß erhitzt. Man engt ein und versetzt das Rohprodukt der Struktur

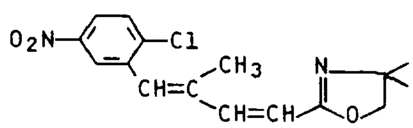

mit 10 ml Wasser und 10 ml konz. HCl. Nach 7-stündigem Erhitzen auf 100 ° C ist der Oxazolinring gespalten. Das Rohprodukt wird abgesaugt, mit Wasser gewaschen und getrocknet. Ausbeute: 7,1 g (66 %) Fp. 178 -185 ° C
4.2 5-(2'-Chlor-5'-nitrophenyl)-4-methyl-2,4-pentadiensäuremethylester
Die nach 4.1 erhaltene Säure wird mit Methanol/HCl verestert; 49 % Ausbeute (Fp.: 87 - 90 ° C).
4.3 5-(2'-Chlor-5'-aminophenyl)-4-methyl-2,4-pentadiensäuremethylester
Die Reduktion der nach 4.2 hergestellten Nitroverbindung gelingt wie unter 3.3 beschrieben. Man erhält 2,9 g (97 %; Fp: 47-50 ° C) des Anilinderivates folgender Struktur:

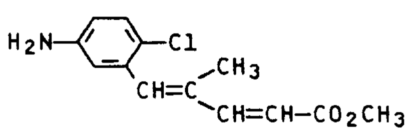

4.4

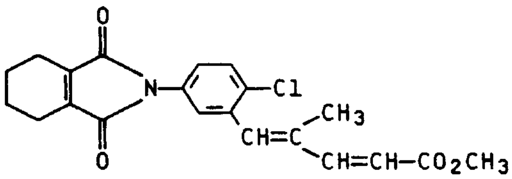

Das nach 4.3 hergestellte Anilinderivat wird mit Tetrahydrophthalsäureanhydrid wie beschrieben in Eisessig umgesetzt. Das Rohprodukt wurde durch Chromatografie an Kieselgel mit Hexan/Aceton = 1 : 1 gereinigt und aus 80 % Methanol umkristallisiert. Man erhielt 1,5 g (35 % d. Th.) des Wirkstoffs (Fp: 160 - 162 °C), der in der Tabelle 4 als Verb. Nr. 4-2 aufgeführt ist. Die Verbindung stellt vermutlich ein Gemisch von Stereoisomeren dar.

Beispiel 5

Zielverbindung:

2-{4-Chlor-3-[(4'-ethoxycarbonyl-3'-methyl-1',3'-butadienyl)-phenyl]}-4,5,6,7-tetrahydro-2H-isoindol-1,3-dion (Wirkstoff 3-3 in Tabelle 3)

5.1 5-(2'-Chlor-5'-nitrophenyl)-2-methyl-2,4-pentadiensäureethylester (Horner-Emmons-Reaktion)
Zu einer Suspension von 0,65 g 80 proz. NaH in 30 ml absolutem Dimethylformamid (DMF) wird eine Lösung von 5,3 g 3-Methyl-3-ethoxycarbonyl-2-propen-1-phosphonsäurediethylester in 10 ml DMF bei 0 - 10 °C zugetropft.
Nach zweistündigem Rühren bei 40 °C ist die Wasserstoffentwicklung abgeschlossen; man tropft nun eine Lösung von 3,7 g 2-Chlor-5-nitro-benzaldehyd in 10 ml DMF zu und läßt 5 Std. bei 60 °C rühren. Anschließend tropft man 5 ml Methanol zu, gibt 20 ml konz. HCl zu, extrahiert 3 mal mit je 100 ml Diethylether und wäscht 1 mal mit Wasser. Nach Einengen und Chromatografie über Kieselgel mit Toluol erhält man 3,8 g (67 % d. Th.; Fp. 80-82 °C) Feststoff. Struktur:

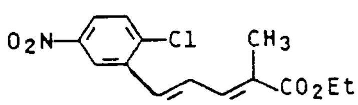

5.2 Reduktion der Nitrogruppe
Die nach 5.1 erhaltene Nitroverbindung wurde mit Eisen in Ethanol/Eisessig reduziert. Man erhielt in 96 % Ausbeute ein gelbliches Öl, das ohne weitere Reinigung zum Wirkstoff umgesetzt wurde.
5.3 1,35 g Tetrahydrophthalsäureanhydrid und 2,35 g des nach 5.2 erhaltenen Anilinderivates wurden wie beschrieben in 25 ml Eisessig umgesetzt. Nach der Aufarbeitung erhielt man 2,1 g (59 % d. Th.) des Wirkstoffs (Fp: 123 - 125 °C), der in Tabelle 3 als Verb. 3-3 aufgeführt ist.

Beispiel 6:

Zielverbindung:

2-{4-Chlor-3-[(4'-methoxycarbonyl-2',4'-dimethyl-1',3'-buta-dienyl-phenyl]} -4,5,6,7-tetrahydro-2H-isoindol-1,3-dion (Wirkstoff 3.4 in Tabelle 3)

6.1 5-(2'-Chlor-5'-nitrophenyl)-2,4-dimethyl-2,4-pentadiensäure (Perkin-Kondensation)
Man gibt 22,6 g 2-Chlor-5-nitro-α-methylzimtaldehyd und 9,6 g Natriumpropionat in 16,3 g Propionsäureanhydrid und läßt das Gemisch 22 Std. bei 140 °C rühren. Nach dem Abkühlen wird das Reaktionsgemisch in 100 ml Wasser gegossen, auf pH 10 gebracht (NaOH) und mit Essigester extrahiert. Nach dem

Ansäuern der wässr. Phase mit konz. HCl wird der ausgefallene Niederschlag abgesaugt, mit Wasser gewaschen und getrocknet. Man erhält 18 g (64 % d. Th.) eines gelben Feststoffs mit Fp. 180 - 182 °C, der folgenden Struktur besitzt:

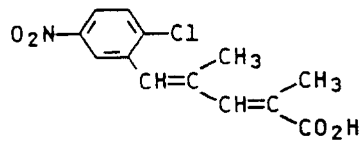

6.2 5-(2'-Chlor-5'-nitrophenyl)-2,4-dimethyl-2,4-pentadiensäuremethylester

Veresterung der Säure mit Methanol/HCl. Man erhielt den Ester in 78 % Ausbeute, Fp. 88 - 90 °C.

6.3 5-(2'-Chlor-5'-aminophenyl)2,4-dimethyl-2,4-pentadiensäure-methylester

Die Nitroverbindung wurde mit Eisen in Methanol/Eisessig reduziert. Man erhielt 12,3 g (95 %) eines gelblichen Öls, das ohne weitere Reinigung weiter verwendet wurde.

6.4 2-{4-Chlor-3-[(4'-methoxycarbonyl-2',4'-dimethyl-1',3'-buta-dienyl)-phenyl]}-4,5,6,7-tetrahydro-2H-isoindol-1,3-dion

12,3 g des nach 6.3 erhaltenen Anilinderivates und 7 g 2,3-Tetramethylen-maleinsäureanhydrid werden 2 Std. in 70 ml Eisessig bei 40 °C gerührt und anschließend zwei Std. zum Sieden erhitzt. Nach Abtrennen des Lösungsmittels am Rotationsverdampfer wird mit n-Hexan/Aceton 9 : 1 über Kieselgel chromatografiert und anschließend einmal aus Methanol umkristallisiert. Man erhält 12,5 g (68 % d. Th.) eines farblosen Feststoffs (Fp: 105 - 107 °C; (Verb. 4-4 in Tabelle 4).

Analog können die übrigen in Tabelle 4 aufgeführten Verbindungen erhalten werden.

Beispiel 7

Zielverbindung:

2-{4-Chlor-3-[(4',4'-bis-methoxy-carbonyl-1'-butenyl)-phenyl]}-4,5,6,7-tetrahydro-2H-isoindol-1,3-dion (Wirkstoff 4-1 in Tabelle 4)

7.1 2-Chlor-5-nitro-cinnamylidenmalonsäure-dimethylester

Zu einer Mischung von 21,7 g Chlor-5-nitrozimtaldehyd und 26,4 g Malonsäuredimethylester in 20 ml DMF gibt man 2 ml Piperidin und erwärmt 12 Std. auf 60 °C. Danach zieht man alle Niedrigsieder bei 0,1 bar und 50 °C Badtemperatur am Rotationsverdampfer ab, gießt in Wasser und verrührt den ausgefallenen Feststoff mit Methanol. Nach Absaugen und Trocknen i. Vak. bei 50 °C erhält man 15,6 g (48 % d. Th.) eines gelben Feststoffs mit Fp. 167 - 168 °C.

7.2 2-Chlor-5-amino-cinnamylmalonsäure-dimethylester

Zu einer Lösung von 4,8 g der nach 7.1 erhaltenen Nitroverbindung in 100 ml Dioxan gibt man 50 ml einer frisch bereiteten gesättigten Lösung von Cu-II-acetat in Methanol. Danach fügt man portionsweise 2 g $NaBH_4$ zu und läßt 4 Std. bei 35 °C rühren. Anschließend wird mit 300 ml Diethylether versetzt und mit $NaHCO_3$-Lsg. extrahiert. Die wässrige Phase wird verworfen, die Etherphase wird getrocknet und eingeengt. Man erhält 4,18 g (95 %) eines Öls, dem die folgende Struktur zukommt:

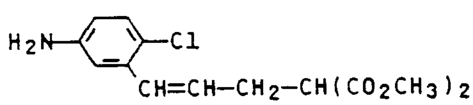

7.3 Tetrahydro-isoindol-1,3-dion-derivat

2,1 g 2,3-Tetramethylen-maleinsäureanhydrid und 4,1 g des nach 7.2 erhaltenen Öls werden in 25 ml Essigssäure 1 Std. bei 40 °C gerührt und danach noch 1 Std. auf 110 °C erhitzt. Nach Abziehen des Solvens wird über Kieselgel mit n-Hexan chromatografiert. Man erhält 3,5 g (58 %) eines Öls ($n^{24}$ = 1,5538) dem die nachstehende Struktur zukommt:

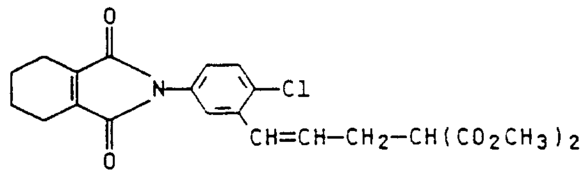

(Verb. Nr. 5-1 in Tabelle 5)

Beispiel 8

Zielverbindung:

2-{4-Chlor-3[(4',4'-bis-methoxycarbonyl-2'-methyl-1'-butenyl)-phenyl]}-4,5,6,7-tetrahydro-2H-isoindol-1,3-dion (Wirkstoff 4-2 in Tabelle 4)

8.1 2-Chlor-5-nitro-α-methylzimtaldehyd
Zu einer Suspension von 111 g 2-Chlor-5-nitrobenzaldehyd in 500 ml Methanol gießt man eine Lösung von 2,4 g NaOH in 20 ml Wasser und kühlt auf 10 °C. Dann tropft man anschließend 42 g Propionaldehyd zu. Es entsteht eine klare Lösung, aus der im Laufe von 1 Std. ein Niederschlag auszufallen beginnt. Nach 16 Std. Rühren bei 20 - 25 °C wird mit Essigsäure auf pH 5 gestellt, der Feststoff abgesaugt und das Filtrat eingeengt. Rückstand und Feststoff werden vereinigt, in Methylenchlorid gelöst und mit Wasser gewaschen. Man trocknet und engt ein. Das Rohprodukt wird mit Petrolether/Ether = 1 : 1 durchgerührt, abgesaugt und getrocknet.
Ausbeute: 109 g (81 %) Fp. 102 - 104 °C
8.2 2-Chlor-5-nitro-α-methyl-cinnamyliden-malonsäuredimethylester
Zu einer Lösung von 26,4 g Malonsäuredimethylester in 20 ml Tetrahydrofuran (THF) gibt man 22,6 g des nach 8.1 erhaltenen Aldehyds und tropft zuletzt eine Lösung von 2 ml Piperidin in 10 ml THF zu. Anschließend erwärmt man 4 Std. auf 60 °C. Nach dem Abkühlen wird das Lösungsmittel i.V. abgezogen, der Rückstand mit Methanol verrührt, abgesaugt und i.V. getrocknet. Man erhält einen gelben Feststoff mit Fp. 100 - 102 °C. Die Ausbeute beträgt 19,3 g (57 %).
8.3 2-Chlor-5-amino-α-methyl-cinnamyl-malonsäuredimethylester
In der gleichen Weise, wie im Beispiel 7.2 beschrieben, wird der bei 8.2 erhaltene Nitro-ester mit $NaBH_4$ in Gegenwart von Kupferacetat in Methanol reduziert. Aus 2,4 g 8.2 erhält man so 1,4 g (63 %) des gewünschten Produkts als Öl. Die Struktur ist:

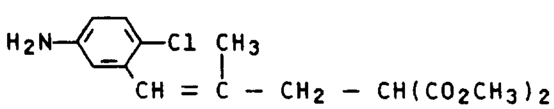

8.4 Tetrahydro-isoindol-1,3-dion-derivat
Das bei 8.3 erhaltene Anilinderivat wird entsprechend dem Vorgehen im Beispiel 7.3 mit 2,3-Tetramethylenmaleinsäureanhydrid in Eisessig umgesetzt. Man erhält so aus 1,4 g Anilinderivat 1,26 g (63 %) des Wirkstoffs mit Fp. 84 - 86 °C, dessen Struktur in Tabelle 5 für die Verbindung 5-2 angegeben ist. In analoger Weise können auch die übrigen Verbindungen aus Tabelle 5 erhalten werden.

Tabelle 1

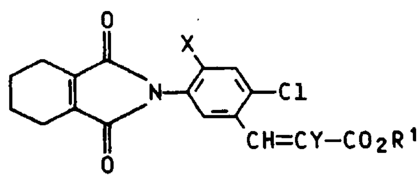

| Lfd.Nr. | X | Y | $R^1$ | Physik.-Konstante |
|---|---|---|---|---|
| 1-1 | F | Br | $CH_3$ | Fp. 121-123°C |
| 1-2 | F | Br | $C_2H_5$ | Fp. 105-110°C |
| 1-3 | F | Cl | $CH_3$ | |
| 1-4 | F | Cl | $C_2H_5$ | |
| 1-5 | F | Br | n-$C_3H_7$ | |
| 1-6 | F | Br | n-$C_4H_9$ | Fp. 55-60°C |
| 1-7 | F | Br | n-$C_5H_{11}$ | |
| 1-8 | F | Br | n-$C_6H_{13}$ | Öl |
| 1-9 | F | Br | cyclo-$C_5H_9$ | |
| 1-10 | F | Br | cyclo-$C_6H_{11}$ | |
| 1-11 | F | Cl | $(CH_2)_2$-$OCH_3$ | |
| 1-12 | F | Cl | $(CH_2)_2$-S-$CH_3$ | |

Tabelle 2

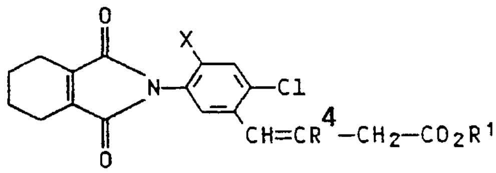

| Nr. | X | $R^4$ | $R^1$ | Physik.-Konstante |
|---|---|---|---|---|
| 2-1 | H | $CH_3$ | $CH_3$ | $n_D^{24}$ = 1,5579 |
| 2-2 | H | $CH_3$ | $C_2H_5$ | |
| 2-3 | H | $CH_3$ | $n-C_4H_9$ | |
| 2-4 | H | $CH_3$ | $-(CH_2)_2OCH_3$ | |
| 2-5 | H | $CH_3$ | $-(CH_2)_2-S-CH_3$ | |
| 2-6 | H | $C_2H_5$ | $CH_3$ | |
| 2-7 | F | $C_2H_5$ | $CH_3$ | |
| 2-8 | H | $C_2H_5$ | $C_2H_5$ | |
| 2-9 | H | $C_2H_5$ | $n-C_4H_9$ | |

Tabelle 3

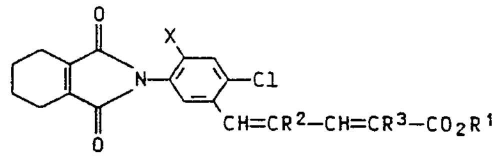

| Nr. | X | $R^2$ | $R^3$ | $R^1$ | Physik.-Konstante |
|---|---|---|---|---|---|
| 3-1 | H | H | H | $CH_3$ | Fp. 127-130°C |
| 3-2 | H | $CH_3$ | H | $CH_3$ | Fp. 160-162°C |
| 3-3 | H | H | $CH_3$ | $C_2H_5$ | Fp. 123-125°C |
| 3-4 | H | $CH_3$ | $CH_3$ | $CH_3$ | Fp. 105-107°C |
| 3-5 | F | H | H | $C_2H_5$ | |
| 3-6 | F | H | H | $n-C_3H_7$ | |
| 3-7 | F | H | H | $n-C_4H_9$ | |
| 3-8 | H | $C_2H_5$ | H | $CH_3$ | |
| 3-9 | H | $CH_3$ | H | $C_2H_5$ | |
| 3-10 | H | $CH_3$ | H | $n-C_4H_9$ | |
| 3-11 | H | $CH_3$ | H | $-(CH_2)_2-OCH_3$ | |
| 3-12 | H | $CH_3$ | H | $-(CH_2)_2-S-CH_3$ | |

Tabelle 4

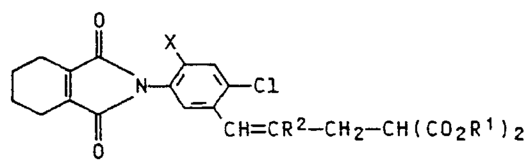

| Nr. | X | $R^2$ | $R^1$ | Physik.-Konstante |
|---|---|---|---|---|
| 4-1 | H | H | $CH_3$ | $n_D^{24}$ = 1,5538 |
| 4-2 | H | $CH_3$ | $CH_3$ | Fp. 84-86°C |
| 4-3 | H | $C_2H_5$ | $CH_3$ | |
| 4-4 | H | H | $C_2H_5$ | |
| 4-5 | H | H | $n-C_3H_7$ | |
| 4-6 | H | H | $n-C_4H_9$ | |
| 4-7 | H | $CH_3$ | $C_2H_5$ | |
| 4-8 | H | $CH_3$ | $n-C_3H_7$ | |
| 4-9 | H | $CH_3$ | $n-C_4H_9$ | |
| 4-10 | H | $CH_3$ | $-(CH_2)_2OCH_3$ | |
| 4-11 | H | $CH_3$ | $-(CH_2)_2OC_2H_5$ | |
| 4-12 | H | $CH_3$ | $-(CH_2)_2O-C_4H_9$ | |

Die Applikation der erfindungsgemäßen Verbindungen als herbizide Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die

unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,005 bis 3,0 vorzugsweise 0,015 bis 0,5 kg/ha.

Die herbizide Wirkung der Isoindoldionderivate der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zweck der Nachauflaufbehandlung zieht man die Testpflanzen je nach Wuchsform erst bis zu einer Wuchshöhe von 3 bis 15 cm an und behandelt sie dann mit den in Wasser als Verteilungsmittel suspendierten oder emulgierten Wirkstoffen, die mittels fein verteilender Düsen gespritzt werden. Es werden direkt gesäte und in den gleichen Gefäßen aufgewachsene Pflanzen ausgewählt, oder sie werden erst als Keimpflanzen getrennt angezogen. Die Aufwandmengen für die Nachauflaufbehandlung betragen 0,015, 0,125 und 0,5 kg Wirkstoff (a.S.)/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 36 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

| Lateinischer Name | Deutscher Name | Englischer Name |
|---|---|---|
| Amaranthus spp. | Fuchsschwanzarten | pigweed |
| Arachis hypogaea | Erdnuß | peanuts (groundnuts) |
| Cassia tora | - | sicklepod |
| Centaurea cyanus | Kornblume | cornflower |
| Chrysanthemum spp. | Wucherblumearten | marigold |
| Galium aparine | Klettenlabkraut | catchweed bedstraw |
| Ipomoea spp. | Prunkwindearten | morningglory |
| Lamium amplexicaule | Stengelumfassende Taubnessel | henbit |
| Mercuralis annua | Einjähriges Bingelkraut | annual mercury |
| Oryza sativa | Reis | rice |
| Sesbania exaltata | Turibaum | hempsesbania (coffeeweed) |
| Solanum nigrum | Schwarzer Nachtschatten | black nightshade |
| Triticum aestivum | Weizen | wheat |
| Veronica spp. | Ehrenpreisarten | speedwell |
| Viola spp. | Stiefmütterchen | violet |
| Zea mays | Mais | Indian corn |

Die Verbindungen Nr. 3-1, 3-2, 3-4 sowie 1-1 entfalten bei Nachauflaufanwendung von 0,5 kg Wirkstoff/ha eine starke herbizide Aktivität gegen breitblättrige unerwünschte Pflanzen.

Die herbizide Wirkung bei Nachauflaufanwendung von 0,125 kg a.S./ha zeigen die Beispielsverbindungen Nr. 4-2, 4-1, 2-1.

Verbindung Nr. 3-2 wirkt bei Nachauflaufanwendung von 0,125 kg a.S./ha gegen breitblättrige Unkräuter selektiv in Weizen, wobei dieser nur anfänglich und temporär leicht geschädigt wird.

Desgleichen bekämpft Verbindung Nr. 3-1 mit 0,125 ha a.S./ha und Nachauflaufbehandlung unerwünschte Pflanzen selektiv in Erdnüssen, wobei diese nur kurzzeitige, sich auswachsende Schäden erleiden.

Unerwünschte breitblättrige Pflanzen in Weizen, Reis und Mais werden mit 0,015 kg a.S./ha der Verbindung Nr. 1-1 erfaßt. An den Kulturen treten höchstenfalls vorübergehende akzeptable Blattschäden auf.

In Anbetracht der Vielseitigkeit der Applikationmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum,Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |

| Botanischer Name | Deutscher Name |
|---|---|
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |

| Botanischer Name | Deutscher Name |
|---|---|
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Tetrahydroisoindoldione der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, (Hetero)-Aryloxy-phenoxypropionsäurederivate, Cyclohexenone und andere in Betracht.

Außerdem kann es von Nutzen sein, die Tetrahydroisoindoldione der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

## Patentansprüche

**1.** Phenylalkenylcarbonsäure und deren Ester der allgemeinen Formel I

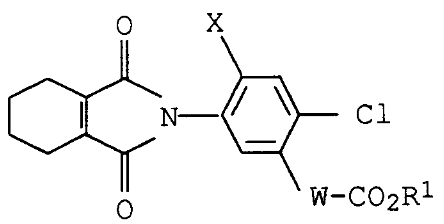

I

in der bedeuten:

X Wasserstoff oder Fluor

$R^1$ Wasserstoff, $C_1$-$C_6$-Alkyl, $C_5$- oder $C_6$-Cycloalkyl, ($C_1$-$C_4$-Alkoxy)-$C_2$-$C_4$-alkyl oder ($C_1$-$C_4$-Alkylthio)-$C_2$-$C_4$-alkyl;

W den zweiwertigen Rest $-CH=CR^4-CH_2-$;
$-CH=CR^2-CH=CR^3-$ oder

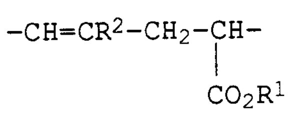

wobei

$R^2$, $R^3$ für Wasserstoff oder $C_1$-$C_4$-Alkyl und $R^4$ für $C_1$-$C_4$-Alkyl stehen und zusätzlich X Fluor und W den Rest $-CH=CY-$ bedeuten, wobei Y für Chlor oder Brom steht.

**2.** Verfahren zur Herstellung von Verbindungen der allgemeinen Formel I gemäß Anspruch 1, dadurch gekennzeichnet, daß man ein Anilin der Formel II mit dem Anhydrid III

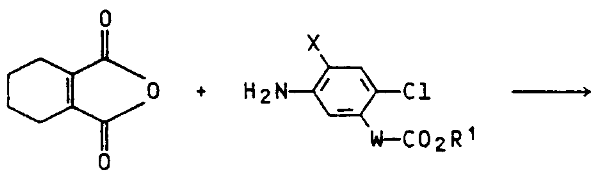 I

III II

in an sich bekannter Weise umsetzt.

3. Zwischenprodukt zur Herstellung von Verbindungen der allgemeinen Formel I gemäß Anspruch 1, nämlich die entsprechende Nitroverbindung der allgemeinen Formel IV

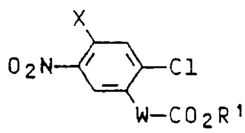 IV

4. Zwischenprodukt zur Herstellung von Verbindungen der allgemeinen Formel I gemäß Anspruch 1, nämlich das entsprechende Anilin der allgemeinen Formel II

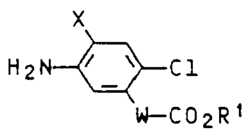 II

5. Herbizides Mittel, enthaltend eine wirksame Menge einer Verbindung der allgemeinen Formel I gemäß Anspruch 1 und übliche Formulierungs- und/oder Verdünnungsmittel.

6. Verfahren zur Bekämpfung von unerwünschtem Pflanzenwuchs, gekennzeichnet durch die Einwirkung einer wirksamen Menge einer Verbindung I gemäß Anspruch 1 auf dessen Lebensraum.

**Claims**

1. A phenylalkenylcarboxylic acid or an ester thereof, of the general formula I

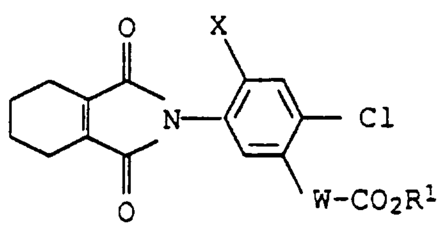 I

where

X is hydrogen or fluorine,

$R^1$ is hydrogen, $C_1$-$C_6$-alkyl, $C_5$- or $C_6$-cycloalkyl, ($C_1$-$C_4$-alkoxy)-$C_2$-$C_4$-alkyl or ($C_1$-$C_4$-alkylthio)-$C_2$-$C_4$-alkyl;

W is a divalent radical -CH = $CR^4$-$CH_2$-, -CH = $CR^2$-CH = $CR^3$-or

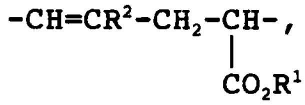

where $R^2$ and $R^3$ are each hydrogen or $C_1$-$C_4$-alkyl and $R^4$ is $C_1$-$C_4$-alkyl, and in addition X is fluorine and W is -CH = CY-, where Y is chlorine or bromine.

2. A process for the preparation of a compound of the general formula I as claimed in claim 1, wherein an aniline of the formula II is reacted in conventional manner with the anhydride III

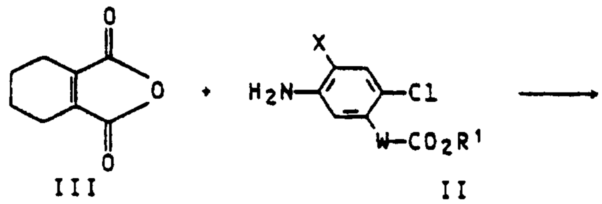

I

III II

3. An intermediate for the preparation of a compound of the general formula I as claimed in claim 1, namely the corresponding nitro compound of the general formula IV

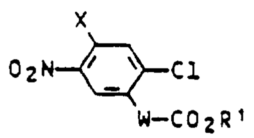

IV

4. An intermediate for the preparation of a compound of the general formula I as claimed in claim 1, namely the corresponding aniline of the general formula II

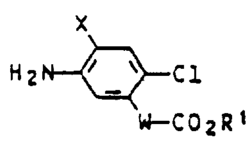

II

5. A herbicidal agent containing an effective amount of a compound of the general formula I as claimed in claim 1 and conventional formulating agents and/or diluents.

6. A method for controlling undesirable plant growth, wherein an effective amount of a compound I as claimed in claim 1 is allowed to act on the plant habitat.

## Revendications

1. Acides phénylalkénylcarboxyliques et leurs esters de la formule générale I

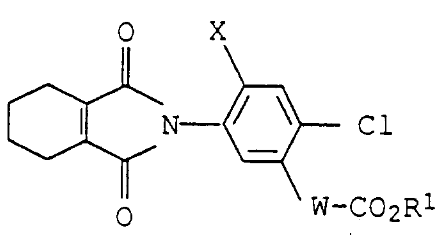

I

dans laquelle

X représente hydrogène ou fluor

$R^1$, hydrogène, alkyle en C1-C6, cycloalkyle en C5- ou C6-, alcoxy en C1-C4, alkyle en C2-C4 ou (alkylthio en C1-C4)-alkyle en C2-C4.

W, le reste bivalent $-CH=CR^4-CH_2-$ ; $-CH=CR^2-CH=CR^3-$ ou

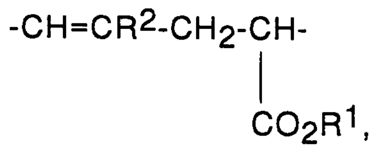

$R^2$, $R^3$ étant mis pour hydrogène ou alkyle en C1-C4 et $R^4$ pour alkyle en C1-C4 et en outre, X représentant fluor et W le reste -CH = CY, Y étant mis pour chlore ou brome.

**2.** Procédé de préparation de composés de la formule générale I selon la revendication 1, caractérisé par le fait qu'on fait réagir, de manière connue en soi, une aniline de formule II avec l'anhydride III

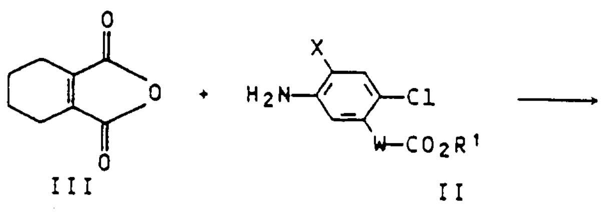

I

III II

**3.** Produit intermédiaire pour la préparation de composés de la formule générale I selon la revendication 1 notamment le composé nitro correspondant de la formule générale IV

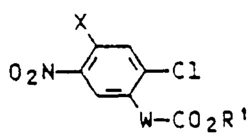

IV

**4.** Produit intermédiaire pour la préparation de composés de la formule générale I selon la revendication 1 notamment l'aniline correspondant à la formule générale II

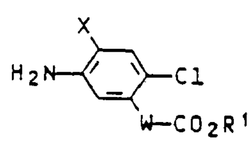

II

**5.** Herbicide contenant une quantité efficace d'un composé de la formule générale I selon la revendication 1 et des agents de préparation et/ou diluants usuels.

**6.** Procédé pour lutter contre la croissance de plantes indésirables caractérisé par le fait que la mise en action d'une quantité efficace d'un composé I selon la revendication 1 sur leur biotope.